# DEMANDE DE BREVET EUROPEEN

(11) **EP 0 645 627 A1**
(43) Date de publication de la demande: **29.03.1995**
(21) Numéro de dépôt: 94114428.9
(22) Date de dépôt: 14.09.1994
(51) Int. Cl.: G01N 33/487, G01N 27/28, C12Q 1/00

(54) **Dispositif de mesure pour capteurs multizones amovibles comprenant un système d'éjection de ces capteurs**

(30) Priorité: 21.09.1993 FR 9311319
(71) Demandeur: ASULAB S.A., CH-2501 Bienne (CH)
(72) Inventeur: Hofmann, Eric, CH-2563 Ipsach (CH); Wicky, André, CH-2300 La Chaux-de-Fonds (CH); Jacquet, Rémy, CH-2400 Le Locle (CH)
(74) Mandataire: Patry, Didier Marcel Pierre

(57) **Abrégé**

La présente invention concerne un dispositif de mesure, notamment pour la mesure du taux de glucose dans le sang, destiné à être associé à un capteur amovible comportant au moins une zone active.

En particulier, l'invention concerne un système d'éjection (138,142,192) servant à éjecter le capteur amovible (108) hors du boîtier (104) du dispositif de mesure. Cette éjection est effectuée lors d'une avance finale du capteur amovible effectuée au moyen d'un dispositif d'avance (110,112,124,130).

## Description

La présente invention concerne un dispositif de mesure destiné à être associé à un capteur amovible comportant une pluralité de zones actives à usage unique. Un tel capteur est nommé par la suite "capteur multizone".

Aux figures 1 et 2 est représenté un dispositif de mesure électrochimique tel que décrit dans la demande de brevet FR 92 01331.

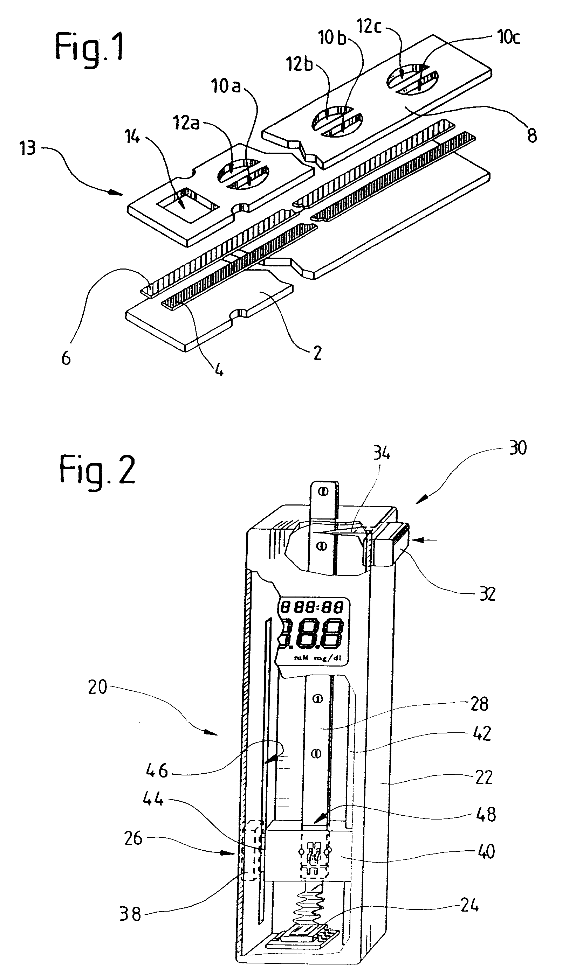

Sur la figure 1 est représenté partiellement et en perspective un éclaté d'un capteur multizone comportant un substrat isolant 2 à la surface duquel sont prévus deux conducteurs 4 et 6 servant à conduire un courant électrique de mesure. Sur ces conducteurs 4 et 6 est prévu un revêtement 8 également isolant.

Le revêtement 8 comporte une première série d'ouverture 10a, 10b, 10c au moins partiellement superposée au premier conducteur 4 et une deuxième série d'ouvertures 12a, 12b, 12c superposée au moins partiellement au deuxième conducteur 6. A une extrémité 13 du revêtement 8 est prévu au moins une ouverture 14 servant à dégager une plage de contact électrique sur chacun des deux conducteurs 4 et 6.

On notera que les conducteurs 4 et 6 sont électriquement isolés l'un de l'autre et que la première série d'ouvertures 10a, 10b, 10c n'est pas superposée au deuxième conducteur 6. De même, la deuxième série d'ouvertures 12a, 12b, 12c n'est pas superposée au premier conducteur 4. Chaque paire d'ouvertures correspondantes 10a et 12a, respectivement 10b et 12b, 10c et 12c définit une zone active du capteur multizone amovible. Le fonctionnement électrochimique d'un tel capteur multizone est sensiblement identique à celui d'un capteur monozone tel que décrit par exemple dans la demande de brevet WO 92/14836.

La substance à analyser, par exemple du sang dans une application possible à la mesure du taux de glucose dans le sang, est apportée dans une des zones actives du capteur multizone. Pour effectuer une mesure électrochimique valable, il est nécessaire que seulement une des zones actives du capteur multizone soit recouverte par la substance à analyser, les zones actives restantes devant nécessairement être non-utilisées.

Sur la figure 2 est représenté un dispositif de mesure électrochimique, désigné par la référence générale 20, destiné à recevoir un capteur multizone amovible 28 du type de celui décrit à la figure 1.

Le dispositif de mesure électrochimique 20 comporte un boîtier 22 à l'intérieur duquel est prévu une électronique de mesure 24. En outre, ce dispositif de mesure électrochimique 20 comprend un dispositif d'avance 26 servant à déplacer le capteur multizone 28 introduit à l'intérieur du boîtier 22.

Afin de couper, après utilisation d'une zone active pour effectuer une mesure électrochimique, le capteur multizone 28 pour séparer la partie de ce capteur comportant cette zone active utilisée du reste de celui-ci, il est prévu un dispositif de coupe 30.

Ce dispositif de coupe 30 comprend un poussoir 32 et une lame 34 solidaire de ce poussoir 32. Le dispositif de coupe 30 est agencé de manière que le poussoir 32 est susceptible d'effectuer une course, selon une direction transversale au capteur multizone 28, suffisamment longue pour permettre à la lame 34 de traverser entièrement ce capteur multizone 28. Le dispositif d'avance 26 comporte un poussoir extérieur 38 solidaire d'un chariot mobile 40 auquel est fixé de manière réversible le capteur multizone 28. Le chariot mobile 40 est susceptible de coulisser le long d'un rail de guidage 42. Le poussoir 38 est relié au chariot mobile 40 au moyen d'une pièce intermédiaire 44 coulissant dans une ouverture rectiligne 46 prévue dans le boîtier 22.

Le dispositif de mesure 20 représenté à la figure 2, bien que de construction simple et peu onéreuse, présente un inconvénient majeur. En effet, si un utilisateur amène le dispositif d'avance 26 et le capteur multizone amovible 28 joint à ce dernier dans une position terminale, c'est-à-dire dans une position où le chariot mobile 40 est situé le plus proche possible du dispositif de coupe 30, et qu'ensuite cet utilisateur actionne ce dispositif de coupe 30 pour couper le capteur multizone amovible 28, la partie terminale 48 de ce capteur multizone 28 reste à l'intérieur du boîtier 22 du dispositif de mesure 20 et il n'est plus possible d'extraire cette partie terminale 48 hors du boîtier 22.

Un but de la présente invention est de pallier l'inconvénient mentionné ci-avant en proposant un dispositif de mesure permettant de sortir entièrement le capteur multizone amovible auquel il est associé et en particulier de sortir la partie terminale de ce capteur multizone amovible dans le cas ou toutes les zones actives ont été séparées de cette partie terminale au moyen d'un dispositif de coupe.

Un autre but de l'invention est de fournir un tel dispositif de mesure permettant de sortir entièrement le capteur multizone amovible hors du boîtier de ce dispositif de mesure sans qu'un utilisateur ait à le saisir manuellement.

Ces buts sont atteints grâce au dispositif de mesure selon l'invention, lequel comprend un boîtier et un connecteur mobile situé à l'intérieur de ce boîtier, ce dernier comportant un orifice pour l'introduction d'un capteur multizone amovible destiné à ce dispositif de mesure. Le connecteur mobile et une partie terminale du capteur multizone amovible comportent respectivement des moyens de fixation complémentaires pour joindre de manière réversible ce connecteur mobile à ce capteur multizone amovible. En outre, ce dispositif de mesure comprend des moyens d'avance pour déplacer le capteur multizone amovible et le connecteur mobile. Ce dispositif de mesure est caractérisé en ce qu'il comprend également des moyens d'éjection servant à éjecter la partie terminale du capteur multizone amovible hors du boîtier de ce dispositif de mesure.

Selon une autre caractéristique de l'invention, la partie terminale du capteur multizone amovible est éjectée hors du boîtier par les moyens d'éjection lors d'une avance finale de ce capteur multizone amovible effectuée par les moyens d'avance prévus dans ce dispositif de mesure.

Selon d'autres caractéristiques particulières de l'invention, les moyens d'éjection comprennent des moyens d'arrêt pour stopper l'avance du connecteur mobile et des moyens de propulsion servant à propulser la partie terminale du capteur multizone amovible hors du boîtier de ce dispositif de mesure.

Selon un mode de réalisation particulier de l'invention, les moyens de propulsion sont formés par une première lame élastique ou ressort plat fixé au boîtier et comprenant une extrémité libre recourbée, cette dernière servant à exercer une force de propulsion sur une face latérale en biseau du capteur multizone amovible lorsque cette face latérale en biseau est située en regard de cette extrémité libre, ladite face latérale en biseau étant située entre une face latérale arrière et une face latérale longitudinale de ce capteur multizone amovible.

D'autres caractéristiques et avantages de l'invention seront décrits ci-après à l'aide de la description suivante faite en référence aux dessins annexés, donnés à titre d'exemples nullement limitatifs, dans lesquels :
- La figure 1, déjà décrite, montre en perspective un éclaté d'un capteur multizone amovible;
- La figure 2, déjà décrite, montre schématiquement un dispositif de mesure associé au capteur multizone amovible représenté à la figure 1;
- La figure 3 est une vue schématique partiellement arrachée d'un mode de réalisation d'un dispositif de mesure selon l'invention;
- La figure 4 est une vue partielle agrandie de la figure 3, mais présentant le capteur multizone amovible associé à ce dispositif de mesure dans une position de mesure finale;
- La figure 5 est une vue en coupe selon la ligne V-V de la figure 4;
- La figure 6 est une vue partielle agrandie de la figure 3, mais représentant le capteur multizone amovible dans une phase d'éjection hors du boîtier du dispositif de mesure;
- La figure 7 est une vue en coupe selon la ligne VII-VII de la figure 6.

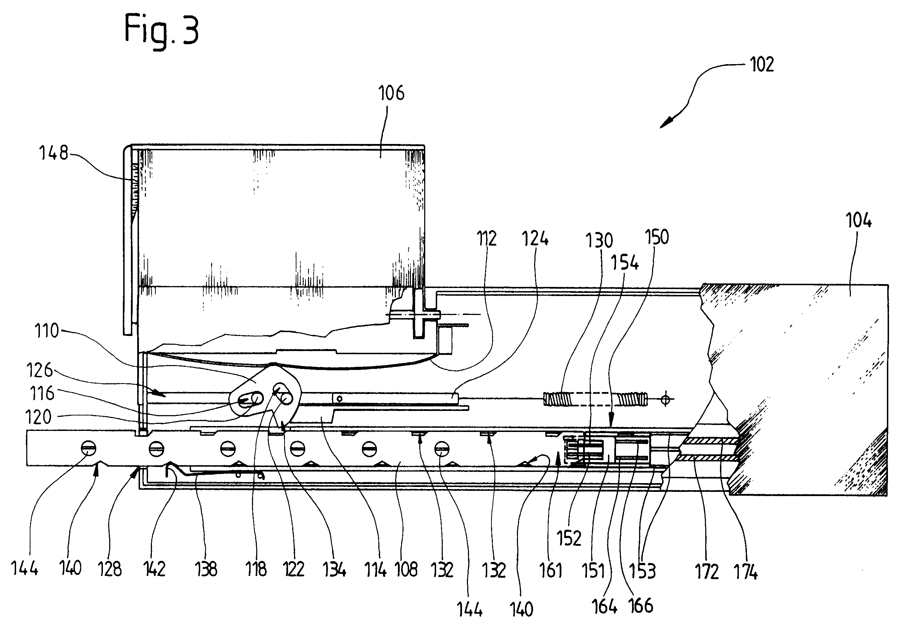

A l'aide des figures 3 à 7, on décrira ci-après un mode de réalisation d'un dispositif de mesure selon l'invention.

Sur la figure 3 est représenté un dispositif de mesure 102 comportant un boîtier 104 dont la partie supérieure a été partiellement arrachée. Le dispositif de mesure 102 est destiné à être associé à un capteur multizone amovible 108. Afin de permettre le déplacement du capteur multizone amovible 108 introduit à l'intérieur du boîtier 104, il est prévu dans ce boîtier 104 un système d'avance comportant une butée mobile 110 associée à une lame élastique ou ressort plat 112 et une butée fixe 114 solidaire du boîtier 104.

La butée mobile 110 comporte deux ouvertures oblongues 116 et 118 dans lesquelles sont respectivement placés deux tétons 120 et 122 faisant saillie d'une barrette mobile 124 guidée dans une coulisse 126. La barrette 124 est reliée solidairement à un poussoir (non représenté) permettant de déplacer la butée mobile 110 depuis sa position de repos, dans laquelle elle est représentée sur la figure 3, en direction de l'orifice 128 servant à l'introduction du capteur multizone amovible 108 à l'intérieur du boîtier 104.

Il est également prévu un ressort de rappel 130 relié à une extrémité à la barrette 124 et à l'autre extrémité au boîtier 104. Ce ressort de rappel 130 permet de ramener la butée mobile 110 dans sa position de repos lorsqu'elle a été déplacée par un utilisateur pour faire avancer le capteur multizone amovible 108 à l'intérieur du boîtier 104.

Le dispositif d'avance est associé à des crans d'avance 132 disposés le long du capteur multizone amovible 108. La butée mobile 110 est agencée de manière que son extrémité 134 s'emboîte dans un des crans d'avance 132 lorsque la butée mobile est actionnée au moyen dudit poussoir par un utilisateur.

Il est également prévu dans le boîtier 104 un ressort de positionnement 138, constitué par une lame élastique ou ressort plat comportant à une extrémité libre un crochet 142 coopérant avec des encoches de positionnement 140 prévues le long du capteur multizone amovible 108. Le ressort de positionnement 138 associé aux encoches de positionnement définit une pluralité de positions de mesure différente du capteur multizone amovible 108 relativement au boîtier 104.

On notera que les encoches de positionnement 140 sont disposées à intervalles sensiblement réguliers le long du capteur multizone amovible 108. De même, les crans d'avance 132 sont disposés le long de ce capteur multizone amovible 108 à intervalles réguliers identiques aux intervalles réguliers prévus entre les encoches de positionnement 140.

La position des encoches de positionnement 140 relativement aux crans d'avance 132 est déterminée en fonction de la position du crochet 142 du ressort de positionnement 138, qui assure le maintien du capteur multizone amovible 108 dans une quelconque position de mesure, et de la butée fixe 114 qui détermine la position de repos de la butée mobile 110.

Dans le cas où le capteur multizone amovible 108 comporte des zones actives 144 à usage unique, il est nécessaire de couper ce capteur multizone amovible 108 après chaque mesure afin de séparer la zone active utilisée pour cette mesure des autres zones actives non-utilisées. Pour ce faire, il est prévu une lame 148 agencée dans le couvercle 106 du dispositif de mesure 102, cette lame 148 subissant un mouvement de rotation solidaire du couvercle 106. Ainsi, il est prévu qu'un utilisateur actionne le couvercle 106 pour couper le capteur multizone 108 entre deux mesures consécutives.

Le capteur multizone 108 est relié électriquement à une électronique de mesure (non représentée) solidaire du boîtier 104 à l'aide d'un connecteur mobile 150 comprenant un chariot mobile 151 guidé dans une coulisse 153 ménagée à l'intérieur du boîtier 104. Le connecteur mobile 150 comprend en outre deux premiers organes de contact électrique 152 et 154 servant à établir respectivement deux contacts électriques avec deux plages de contact électrique respectives 160 et 162 prévues sur une partie terminale 161 du capteur multizone amovible 108. Le connecteur mobile 150 comporte également deux deuxièmes organes de contact électrique 164 et 166 reliés électriquement et respectivement aux deux premiers organes de contact électrique 152 et 154.

Les deux deuxièmes organes de contact électrique 164 et 166 sont constitués, par exemple, par deux lames métalliques élastiques agencées de manière à ce qu'elles exercent respectivement une pression sur deux collecteurs fixes 172 et 174 prévus à l'intérieur du boîtier 104. Ces deux collecteurs fixes 172 et 174, constitués dans ce mode de réalisation par deux pistes métallisées continues, forment en association avec les deux deuxièmes organes de contact électrique respectifs 164 et 166 deux contacts électriques glissants assurant ainsi une connexion électrique fiable entre le connecteur mobile 150 relié électriquement au capteur multizone amovible 108, et un circuit électronique de mesure non représenté.

Les contacts électriques entre les deux premiers organes de contact électrique 152 et 154 et les deux plages de contact électrique respectives 160 et 162 du capteur multizone amovible 108 sont réalisés par les extrémités libres des deux premiers organes de contact électrique 152 et 154, ces extrémités libres étant recourbées. De ce fait, l'introduction du capteur multizone amovible 108 à l'intérieur du boîtier 104 et sa fixation au connecteur mobile 150 sont réalisés sans endommager les deux premiers organes de contact électrique 152 et 154. De même, l'agencement des contacts électriques entre le connecteur mobile 150 et le capteur multizone amovible 108 permet de retirer ce dernier du dispositif de mesure et en particulier du connecteur mobile 150 sans intervention particulière sur ces contacts électriques, si ce n'est l'application d'une force servant à libérer les deux premiers organes de contact électrique 152 et 154 des plages de contact électrique 160 et 162 correspondantes.

Afin de fixer de manière réversible le capteur multizone amovible 108 au connecteur mobile 150, il est prévu un crochet 178 associé à une ouverture 180 prévue dans la partie terminale 161 du capteur multizone amovible 108, le crochet 178 étant formé par une extrémité libre recourbée d'une lame métallique ou ressort plat fixé au chariot 151.

L'agencement du chariot 151 et de la coulisse 153 dans lequel il est susceptible de coulisser est prévu de manière que le crochet 178 est susceptible de subir un déplacement perpendiculaire au plan général du capteur multizone amovible 108 défini par sa surface inférieure 182.

On notera encore que le chariot mobile 151 comporte deux rails de guidage 183 et 184 qui coopèrent avec la coulisse 153 pour guider ce chariot mobile 151 à l'intérieur du boîtier 104.

Le dispositif de mesure selon l'invention comporte des moyens d'éjection de la partie terminale 161 du capteur multizone amovible 108. L'éjection de cette partie terminale 161 par les moyens d'éjection est réalisée lors d'une avance finale du capteur multizone amovible 108 effectué par les moyens d'avance décrits ci-avant.

Les moyens d'éjection susmentionnés comportent premièrement un cran d'avance 190 prévu sur un côté latéral du chariot 151 situé du côté de la butée mobile 110 relativement à ce chariot mobile 151. Ce cran d'avance 190 a pour fonction de permettre un mouvement solidaire de la butée mobile 110 et du chariot mobile 151 lors d'une avance finale de ce chariot mobile 151. Ainsi, lors de cette avance finale, les moyens d'avance sont reliés au chariot mobile 151 et non au capteur multizone amovible.

Le capteur multizone amovible 108 comporte dans sa partie terminale 161 une face latérale en biseau 192 située entre la face arrière 194 et la face latérale longitudinale 196 de ce capteur multizone amovible 108.

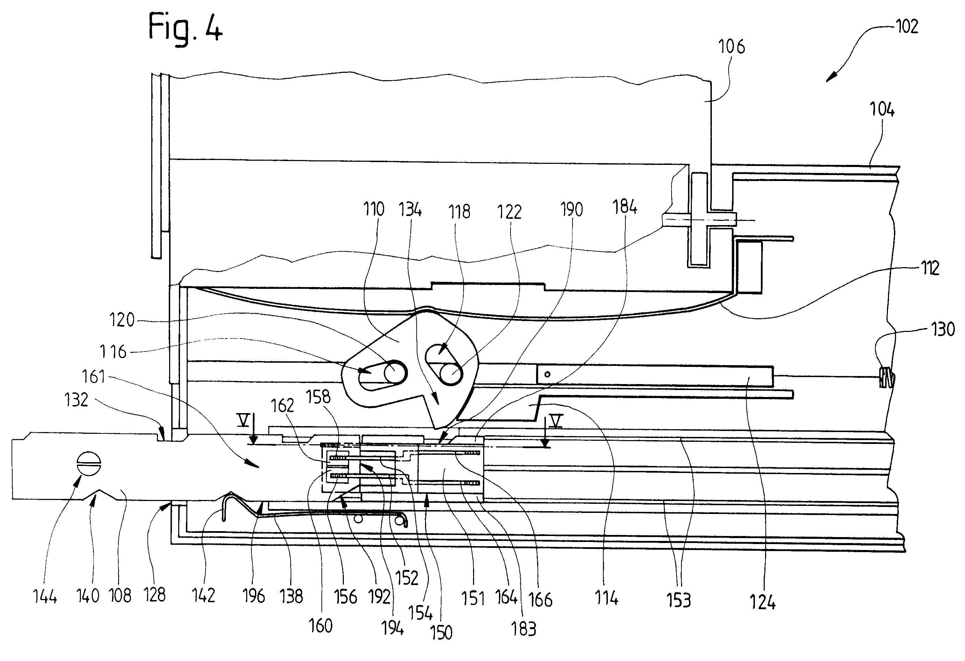

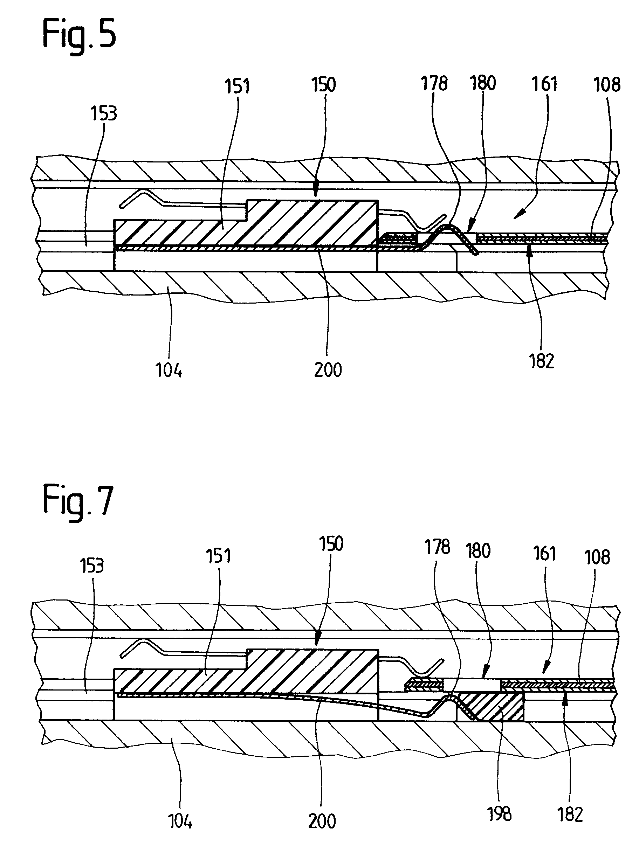

Sur les figures 4 et 5, le connecteur mobile 150 joint à la partie terminale 161 du capteur multizone amovible 108 est représenté dans une position de mesure finale, c'est-à-dire dans une position prévue pour l'utilisation de la dernière zone active 144 représentée à la figure 4. Dans cette position de mesure finale, la butée mobile 110 est située dans sa position de repos, l'extrémité 134 de cette butée mobile étant située en regard du cran d'avance 190 prévu sur la face latérale susmentionnée du chariot mobile 151.

Dans la position de mesure finale susmentionnée, le crochet 178, servant au maintien du capteur multizone amovible 108 au chariot mobile 151, est inséré à l'intérieur de l'ouverture 180 prévue dans la partie terminale 161 du capteur 108, comme dans toute autre position de mesure.

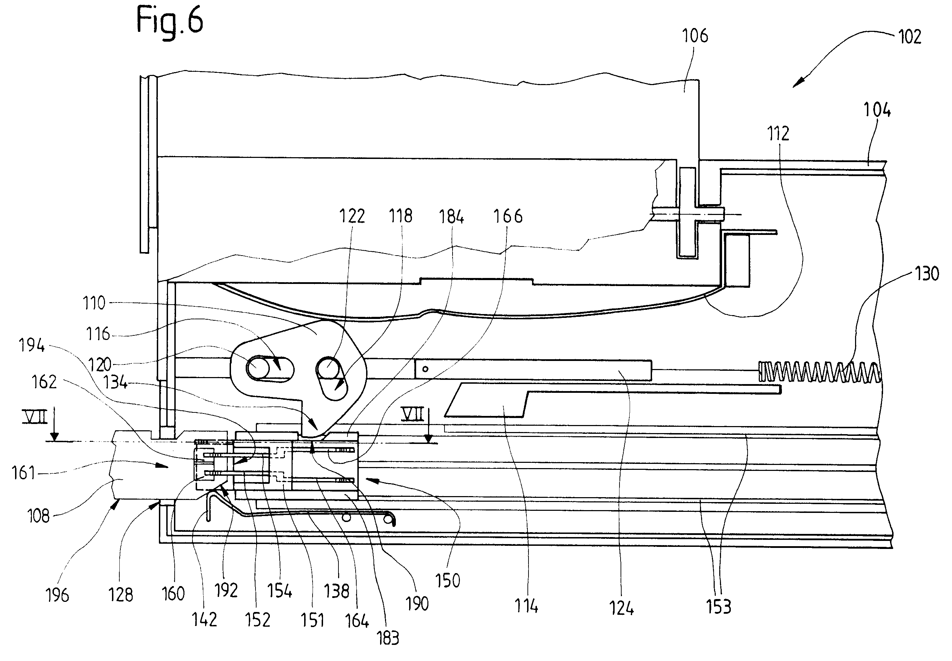

Afin d'éjecter la partie terminale 161 du capteur 108 hors du boîtier 104 du dispositif de mesure 102, la butée mobile 110 est mise en action par un utilisateur. Lorsque cet utilisateur actionne le poussoir mentionné précédemment, la butée mobile 110, associée à la lame élastique 112, subit une rotation de manière que l'extrémité 134 de cette butée mobile 110 vient s'emboîter dans le cran d'avance 190 du chariot mobile 151. Ensuite, l'utilisateur, en continuant d'actionner le système d'avance entraîne en déplacement le chariot mobile 151 joint au capteur multizone amovible 108 jusqu'à ce que ces derniers se trouvent dans la position décrite aux figures 6 et 7 sur lesquelles le chariot mobile 151 est représenté dans sa position terminale et la partie terminale 161 du capteur 108 est représentée dans la phase d'éjection de celle-ci hors du boîtier 104.

Lors de la phase d'éjection, le crochet 142 du ressort de positionnement 138, constitué par une lame élastique dont l'extrémité libre est recourbée, appuie contre la face latérale en biseau 192 de la partie terminale 161 du capteur 108. L'agencement du ressort de positionnement 138, en particulier de son crochet 142, et la face latérale en biseau 192 du capteur 108 permet à ce ressort de positionnement 138 d'exercer une force de propulsion sur la partie terminale 161 du capteur 108. Conjointement, comme cela est représenté à la figure 7, le chariot mobile 151 est stoppé par une butée oblique 198, solidaire du boîtier 104, située en fin de parcours de ce chariot mobile 151.

La butée oblique 198 est agencée de telle manière que le crochet 178, servant à la fixation du capteur multizone amovible 108 au connecteur mobile 150, est dégagé de l'ouverture correspondante 180 prévue dans la partie terminale 161 de ce capteur 108 grâce à une force d'avance exercée par l'utilisateur du dispositif de mesure selon l'invention lors de l'avance finale décrite ici. On notera que le crochet 178 est relié à une lame élastique 200 fixée au chariot mobile 151.

La butée oblique 198 stoppe ainsi le chariot mobile 151, et par conséquent le connecteur mobile 150, pour permettre au ressort de positionnement 138, utilisé ici comme moyens de propulsion de la partie terminale 161 du capteur 108, de vaincre les forces de frottement résultant de la pression exercée par les deux premiers organes de contact électrique 152 et 154 du connecteur mobile 150 sur les plages de contact électrique 160 et 162 correspondantes du capteur 108.

Ainsi, lors d'une avance finale effectuée par un utilisateur, le capteur 108 est libéré du connecteur mobile 150 au moyen de la butée oblique 198 et est ensuite éjectée hors du boîtier 104 par le ressort de positionnement 138 associé à la face latérale en biseau 192 de la partie terminale 161 du capteur amovible 108.

A l'aide du seul poussoir servant à l'avance du capteur multizone amovible 108 pour effectuer diverses mesures consécutives, un utilisateur peut éjecter la partie terminale 161 de ce capteur 108. De ce fait, il est possible de trancher le capteur multizone amovible 108 après chaque mesure effectuée à l'aide de ce capteur 108 et d'éjecter finalement la partie terminale 161 du capteur 108 ne comportant plus aucune zone active hors du boîtier 104 de manière à pouvoir introduire ensuite un nouveau capteur amovible pour effectuer d'autres mesures à l'aide du dispositif de mesure 2 selon l'invention.

## Revendications

1. Dispositif de mesure comportant un boîtier (104) et un connecteur mobile (150) situé à l'intérieur dudit boîtier, ce dernier comportant un orifice (128) pour l'introduction d'un capteur multizone amovible (108) destiné à ce dispositif de mesure, ledit connecteur mobile comportant des moyens de fixation (178,200) pour joindre de manière réversible ce connecteur mobile audit capteur multizone amovible, ledit dispositif de mesure comprenant en outre des moyens d'avance (110,112,124) pour déplacer ledit capteur multizone amovible et ledit connecteur mobile, ce dispositif de mesure étant caractérisé en ce qu'il comprend également des moyens d'éjection (138,142,192,198) servant à éjecter une partie terminale (161) dudit capteur multizone amovible (108) hors dudit boîtier (104).

2. Dispositif de mesure selon la revendication 1, caractérisé en ce que l'éjection de ladite partie terminale (161) dudit capteur multizone amovible (108) hors dudit boîtier (104) est effectué par lesdits moyens d'éjection (138,142,192,198) lors d'une avance finale dudit capteur multizone amovible effectuée par lesdits moyens d'avance (110,112,124).

3. Dispositif de mesure selon la revendication 2, caractérisé en ce que lesdits moyens d'éjection (138,142,192,198) comprennent des moyens d'arrêt (198) pour stopper l'avance dudit connecteur mobile (150) et des moyens de propulsion (138,142) servant à propulser ladite partie terminale (161) dudit capteur multizone amovible (108) hors dudit boîtier (104).

4. Dispositif de mesure selon la revendication 3, caractérisé en ce que lesdits moyens de propulsion (138,142) sont formés par une première lame élastique (138) fixée audit boîtier (104) et comprenant une extrémité libre (142), cette dernière servant à exercer une force de propulsion sur une face latérale en biseau (192) dudit capteur multizone (108) lorsque cette face latérale en biseau est située en regard de cette extrémité libre recourbée, ladite face latérale en biseau (192) étant située entre une face latérale arrière (194) et une première face latérale longitudinale (196) dudit capteur multizone amovible (108).

5. Dispositif de mesure selon la revendication 4, caractérisé en ce que ladite première lame élastique (138) comprend à son extrémité libre un crochet (142) servant, conjointement à un ensemble d'encoches de positionnement (140) prévues le long de ladite première face latérale longitudinale (196) dudit capteur multizone amovible (108), à maintenir ce capteur multizone amovible dans une quelconque position parmi une pluralité de positions différentes déterminées par lesdites encoches de positionnement (140).

6. Dispositif de mesure selon la revendication 4 ou 5, caractérisé en ce que lesdits premiers moyens de fixation (178,200) sont formés par une deuxième lame élastique (200) fixée audit connecteur mobile (150), cette deuxième lame élastique comprenant à une extrémité libre un crochet (178) ayant sensiblement la forme d'un V et étant destiné à être associé à une ouverture de fixation (180) prévue dans ladite partie terminale (161) dudit capteur multizone amovible (108).

7. Dispositif de mesure selon la revendication 6, caractérisé en ce que lesdits moyens d'arrêt (198) sont formés par une butée oblique (198) agencée de manière à ce que ledit crochet (178) de ladite deuxième lame élastique (200), lors de ladite avance finale, vient buter contre cette butée oblique et soit ensuite dégagé de ladite ouverture de fixation (180) sous l'action d'une force exercée par lesdits moyens d'avance (110,112,124).

8. Dispositif de mesure selon l'une des revendications 4 à 7, caractérisé en ce que lesdits moyens d'avance (110,112,124) sont formés par une butée mobile (110) actionnée par un poussoir, cette butée mobile étant destinée à être associée à des crans d'avance (132) prévus le long d'une deuxième face latérale longitudinale dudit capteur multizone amovible (108) et également à un cran d'avance (190) prévu sur un côté latéral dudit connecteur mobile (150), ce côté latéral étant situé sensiblement dans le prolongement de ladite deuxième face latérale longitudinale.

9. Dispositif de mesure selon la revendication 8, caractérisé en ce que ladite butée mobile (110) est associée audit cran d'avance (190) prévu sur ledit côté latéral dudit connecteur mobile (150) lors de ladite avance finale de ce connecteur mobile.

10. Dispositif de mesure selon l'une quelconque des revendications précédentes, caractérisé en ce qu'il comprend un dispositif de coupe (148) servant à trancher ledit capteur multizone amovible (108) pour séparer, après une utilisation d'une zone active (144) de ce capteur multizone amovible, une partie de ce dernier comportant cette zone active.

11. Dispositif de mesure selon l'une quelconque des revendications précédentes, caractérisé en ce que ce dispositif de mesure et ledit capteur multizone amovible (108) auquel il est associé sont agencés pour mesurer le taux de glucose dans le sang.
